(19) Europäisches Patentamt
European Patent Office
Office européen des brevets

(11) Publication number: **0 108 437**
**B1**

(12) **EUROPEAN PATENT SPECIFICATION**

(45) Date of publication of patent specification: **14.05.86**

(51) Int. Cl.$^4$: **C 07 C 69/16,** C 07 C 67/293, C 07 C 67/37

(21) Application number: **83201476.5**

(22) Date of filing: **14.10.83**

(54) **Process for the preparation of ethylidene diacetate and/or acetic acid anhydride.**

(30) Priority: **01.11.82 GB 8231166**

(43) Date of publication of application:
**16.05.84 Bulletin 84/20**

(45) Publication of the grant of the patent:
**14.05.86 Bulletin 86/20**

(84) Designated Contracting States:
**BE DE FR GB IT NL**

(56) References cited:
**EP-A-0 035 860**
**FR-A-2 303 788**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 108 437 B1

**Description**

The present invention relates to a process for the preparation of ethylidene diacetate and/or acetic acid anhydride by carbonylation of methyl acetate and/or dimethyl ether in the presence of a homogeneous Group VIII noble metal catalyst.

Ethylidene diacetate and acetic acid anhydride are valuable chemicals which can be used as such for example as solvent or as intermediates for the preparation of chemicals. Ethylidene diacetate can be converted into vinyl acetate, ethyl acetate or acetic acid anhydride whereas acetic acid anhydride in its turn can be converted into ethylidene diacetate. The most important application of acetic acid anhydride is the use in the manufacture of cellulose acetate.

In the search for suitable processes utilizing synthesis gas for the production of highly valuable chemicals it has previously been proposed to prepare ethylidene diacetate and acetic acid anhydride by carbonylation reactions catalyzed by Group VIII noble metal based catalysts. UK Patent Specification 1,538,782 discloses a process for the preparation of ethylidene diacetate by carbonylation of methyl acetate or dimethyl ether in the presence of hydrogen using a catalytic system comprising a Group VIII noble metal compound, a bromide and/or iodide source, an inorganic promoter such as for example a Group $IV_b$ metal (compound) or a Group $VI_b$ metal (compound), and/or an organic promoter such as for example a tertiary amine or phosphine. The presence of a promoter is considered essential as is demonstrated in the Examples V and VIII.

Processes for the preparation of acetic acid anhydride by carbonylation of methyl acetate using a Group VIII noble metal catalyst are described in UK Patent Specifications 1,468,940, 1,523,346 and 1,538,783. In UK Patent Specification 1,538,783 a catalytic system for this carbonylation reaction is described consisting of a Group VIII noble metal, a bromide or iodide, and a multiple promoter comprising at least one metal of Groups $IV_b$, $V_b$ and $VI_b$, or a non-noble metal of Group VIII, and an organic compound of trivalent nitrogen or phosphorus.

Recently it has been proposed to enhance the activity of Group VIII noble metal catalysts by using specific combinations of metal compounds as promoter. According to German Offenlegungsschrift 2,941,232 the activity of the Group VIII noble metal catalyst in the preparation of ethylidene diacetate from methyl acetate can be increased by carrying out the reaction in the presence of (an) iodine (compound), a heterocyclic aromatic compound having a quaternary nitrogen atom, an aliphatic carboxylic acid and a rhenium or manganese compound. In European Patent Application 0026,280 a similar multiple promoter system is proposed for the preparation of acetic acid anhydride by carbonylation of methyl acetate using, however, a zirconium compound instead of a rhenium or manganese compound.

The Dutch laid open Patent Applications 8,006,806 and 8,006,807 describe Group VIII noble metal catalyst systems having enhanced activity for the carbonylation of methyl acetate to acetic acid anhydride containing an iodide, a trivalent organo nitrogen, phosphorus or arsenic compound in combination with metallic zirconium or hafnium.

It has now surprisingly been found that by carrying out the carbonylation of methyl acetate and/or dimethyl ether in the presence of certain compounds of elements of Group $V_a$ which will be specified below either in the absence or presence of other (known) promoters, the activity of the Group VIII noble metal catalyst can be considerably increased. The Group $V_a$ compounds are oxides, sulfides or selenides of organic Group $V_a$ compounds, in which the Group $V_a$ element has a valency above 3.

The present invention therefore relates to a process for the preparation of ethylidene diacetate and/or acetic acid anhydride by reacting methyl acetate and/or dimethyl ether with carbon monoxide or a mixture of carbon monoxide and hydrogen at elevated temperature and pressure in the presence of a homogeneous catalyst system comprising a Group VIII noble metal compound, a bromide or iodide source, and a Group $V_a$ compound represented by the general formula

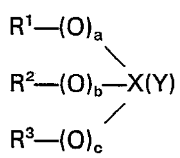

I

in which X is a Group $V_a$ element having a valency above 3 selected from N, P, As or Sb; Y is a Group $VI_a$ element selected from O, S or Se and either a, b and c are 0 or 1 and $R^1$, $R^2$ and $R^3$ are similar or dissimilar optionally substituted hydrocarbon groups; or a and b are 0 and c is 0 or 1 and $R^1$ and $R^2$ form together with X a heterocyclic group or a, b and c are 0 and $R^1$, $R^2$ and $R^3$ form together with X a heterocyclic aromatic group.

Normally the process of the invention will be carried out using methyl acetate as the starting material. It is also possible to use dimethyl ether or a mixture thereof with methyl acetate as starting material. It would appear that dimethyl ether will be converted under reaction conditions into methyl acetate by introduction of a CO moiety. If desired the process can be carried out in two or more stages when dimethyl ether is used as starting material. Firstly dimethyl ether will be (partially) converted into methyl acetate,

which in its turn, in the same or in a different vessel, will be converted into the final products ethylidene diacetate and/or acetic acid anhydride.

It should be noted that the overall reactions when methyl acetate and dimethyl ether are converted into ethylidene diacetate and acetic acid anhydride can be expressed by the following chemical equations

(1) 2MA+2 CO+H2→EDA+AA
(2) DME+4 CO+H2→EDA+AA
(3) MA+CO→AAA
(4) DME+2 CO→AAA

in which AA=acetic acid, AAA=acetic acid anhydride, DME=dimethyl ether, EDA=ethylidene diacetate and MA=methyl acetate. Acetic acid appears to be the primary by-product but other compounds, for example acetaldehyde may also be produced. While the mechanism of the reactions occurring is not known it is not possible to indicate all factors which govern the composition of the reaction mixture. However, the amount of hydrogen present seems to influence this composition to a substantial measure in that higher amounts of hydrogen do increase the amount of ethylidene diacetate formed. It should be noted, however, that this does not necessarily mean that the ethylidene diacetate is formed via hydrogenation of the acetic acid anhydride formed by carbonylation.

The Group VIII noble metal compounds which can be used in the process of the invention include especially rhodium and palladium compounds although other Group VIII noble metal compounds can also be used. Examples of rhodium compounds which may be used include rhodium oxide, rhodium (III) hydroxide, rhodium (III) chloride, rhodium (III) chloride trihydrate, rhodium (III) bromide, rhodium (III) iodide and the corresponding pyridine and phosphine complexes such as tris(pyridine) rhodium (III) chloride or dichloro bis(triphenylphosphine) rhodium, rhodium (III) formate, rhodium (III) acetate, rhodium (III) butyrate, rhodium (III) naphtenate, dirhodium octacarbonyl, tetrarhodium dodecacarbonyl, hexarhodium hexadecacarbonyl, rhodium dicarbonyl acetylacetonate and other organo rhodium complexes. Preference is given to the use of rhodium (III) chloride trihydrate.

Examples of palladium compounds which may be used include palladium chloride, palladium chloride dihydrate, palladium bromide, palladium iodide, palladium oxide and organic palladium salts or complexes such as palladium formate, palladium acetate, palladium butyrate and palladium acetylacetonate. Preferred palladium compounds are palladium chloride, palladium chloride dihydrate and palladium acetate.

In European Patent Application 0035860 a process for the preparation of ethylidene diacetate and/or acetaldehyde by carbonylation of methyl acetate or dimethyl ether has been described. The reaction is carried out in the presence of a catalyst consisting of palladium metal supported on a porous inorganic material, a halide and optionally a promoter. It is stated above that a catalyst consisting of metallic palladium, a halide and a promoter did not have catalytic activity, or in other words that the presence of a porous inorganic support is essential. As possible promoters a huge amount of nitrogen compounds, including pyridine oxide, are mentioned, but compounds of trivalent nitrogen are clearly preferred. It cannot be deduced from European Patent Application 0035860 that compounds having the above general formula I can advantageously be used as promoter in a process in which instead of palladium metal on an inorganic support a soluble Group VIII noble metal compounds are used as catalysts.

The amount of Group VIII noble metal to be used in the process of the invention is not critical and any amount which exerts catalytic activity can be used. Amounts as low as 0.001 %w calculated on methyl acetate and/or dimethyl ether to be converted can be used, preference being given to amounts in the range from 0.005—10 %w, most preferably between 0.01—5 %w.

Any iodide or bromide source or mixtures thereof may be used in the process according to the present invention. Suitable iodide and/or bromide sources are elemental iodine, elemental bromine, hydrogen iodide, hydrogen bromide and metal iodides or bromides. Examples of metal iodides or bromides comprise iodides or bromides of alkali metals, Group II metals and transition metals such as lithium iodide, sodium iodide, magnesium iodide, chromium (III) iodide, cobalt (II) iodide, cobalt (II) bromide, nickel (II) iodide, nickel (II) bromide, copper (II) iodide, copper (II) bromide, zinc iodide and zinc bromide. Combinations of metal salts which can give rise to in situ formation of iodides or bromides of Group II metals and transition metals can also be used, for example a mixture of zinc acetate and an alkali (ne earth) metal iodie or bromide. Further bromide or iodide sources which can be used conveniently comprise organic compounds having the general formula $R(CO)_n$ Hal wherein n=0 or 1, Hal represents Br or J and R represents an alkyl group having from 1 to 12 carbon atoms or an aryl, aralkyl or alkaryl group having up to 12 carbon atoms. The use of iodide sources is preferred, in particular alkyl iodides or alkanoyl iodides. Most preference is given to the use of methyl iodide. The quantity of the iodide or bromide source added to the reaction mixture is not crucial. Suitably the number of moles of iodide and/or bromide source per gram atom of Group VIII noble metal is in the range of from 0.1:1 to 1000:1 preferably 1:1 to 500:1 and especially 10:1 to 300:1.

As stated hereinbefore the process according to the present invention is carried out in the presence of a Group $V_a$ compound having the formula (I). The optionally substituted hydrocarbon groups represented by $R^1$, $R^2$ and $R^3$ may be alkyl, cycloalkyl, aryl or alkaryl groups containing up to 30 carbon atoms. When $R^1+R^2$

or $R^1+R^2+R^3$ with X form a heterocyclic group, the hydrocarbon moiety of the heterocyclic group may contain up to 20 carbon atoms.

Preferred Group $V_a$ compounds are represented by the general formula I in which a, b and c are 0, X is P, Y is O or S and $R^1$, $R^2$ and $R^3$ are alkyl groups containing 1—4 carbon atoms or cycloalkyl, aryl or alkaryl groups containing 5—12 carbon atoms. Group $V_a$ compounds of this type having the general formula I in which Y is O are generally preferred.

Other preferred $V_a$ compounds are represented by the general formula I in which a, b and c are 0, X is N, Y is O and $R^1$, $R^2$ and $R^3$ are alkyl groups containing 1—12 carbon atoms or cycloalkyl, aryl or alkaryl groups containing 5—12 carbon atoms or $R^1$ and $R^2$ form together with X a heterocyclic group containing 4—12 carbon atoms; or $R^1$, $R^2$ and $R^3$ form together with X a heterocyclic aromatic group containing 5—12, preferably 5—7 carbon atoms. The optional substituents of the hydrocarbon groups should be substantially inert in the reaction medium. Suitable substituents are for example chlorine, alkoxy groups, carboxylic acid (ester) groups or sulphone or sulphoxide groups.

Examples of suitable Group $V_a$ compounds having formula I are oxides of tertiary amines such as trimethylamine oxide, triethylamine oxide, N,N-dimethyl phenylamine oxide, N,N-dimethyl p-methylphenylamine oxide, N-methyl piperidine oxide, dimethyl octylamine oxide and dimethyl dodecylamine oxide; oxides, sulfides or selenides of tertiary phosphines, arsines or stibines such as trimethylphosphine oxide, triethylphosphine oxide, tri-n-butylphosphine oxide, triphenylphosphine oxide, tri-p-tolylphosphine oxide, tricyclohexylphosphine oxide, diphenyl ethylphosphine oxide, tris(1-naphthyl)phosphine oxide, 1-phenylphospholane oxide, 1-phenylphosphorinane oxide, trimethylphosphine sulfide, tri-4-chlorophenylphosphine sulfide, triphenylphosphine sulfide, tricyclohexylphosphine sulfide, tri-n-butylphosphine sulfide, triphenylphosphine selenide, tris(1-naphtyl)phosphine selenide, triethyl arsine oxide, triphenyl arsine oxide, triphenyl stibine oxide and triphenyl arsine sulfide. Triphenylphosphine oxide and triphenylphosphine sulfide are preferred.

Further examples of suitable Group $V_a$ compounds having formula I comprise alkyl and aryl esters of phosphoric, phosphonic and phosphinic acids and their arsenic or antimony analogues such as trimethyl phosphate, triethyl phosphate, tri-n-butyl phosphate, triphenyl phosphate, dimethyl methyl phosphonate, 1,5-dimethylbicyclo(3.2.1)octyl octyl phosphonate, diethyl methyl phosphonate and methyl diphenyl phosphonate. Preference is given to the use of phosphonates. Still further examples of suitable Group $V_a$ compounds having formula I are oxides of aromatic heterocyclic N-compounds such as pyridine-N-oxide, quinoline-N-oxide, isoquinoline-N-oxide, 1,10-phenantroline-N-monoxide, pyrazine-N-monoxide and the alkyl substituted derivatives thereof for example alpha-picoline-N-oxide. Pyridine-N-oxide is preferred.

It should be noted that the Group $V_a$ compounds having formula I may contain two or more moieties represented by the general formula I. Examples of such compounds are N,N,N',N'-tetramethylethylene diamine-N,N'-dioxide, tetraphenyl trimethylene diphosphine dioxide, tetraphenyl trimethylene diphosphine dioxide, 2,2'-bipyridyl-N,N'-dioxide, 1,10 phenanthroline-N,N'-dioxide and pyrazine-N,N'-dioxide.

It will be appreciated that in the reaction mixture salts or complexes may be formed by the reaction of the oxide, sulfide or selenide of the tertiary N, P, As or Sb compounds with the iodine or bromine compound present or with hydrogen bromide or hydrogen iodide formed therefrom in situ. Examples of such salts and complexes are alkoxy pyridinium salts for example methoxy pyridinium iodide from pyridine oxide and methyl iodide and the complexes

$$[(C_6H_5)_3PO—H—OP(C_6H_5)_3]^+I_3^- \text{ or } [(C_2H_5)_3AsO—H—OAs(C_2H_5)_3]^+I^-.$$

Consequently the use of such salts or complexes when prepared separately is within the scope of the present invention.

Furthermore, it will be appreciated that the oxides of the Group $V_a$ compounds having formula I which are oxides can be formed in situ. For example a phosphine oxide can be formed in situ from a phosphine by carrying out the reaction in the presence of molecular oxygen or hydrogen peroxide.

The amount of the Group $V_a$ compound having formula I to be used in the process of the invention is not critical and may vary from 0.01:1 to 200:1 mol Group $V_a$ compound per gram atom Group VIII noble metal.

As stated hereinbefore the Group $V_a$ compound having formula I can be used either in the absence or presence of other (known) promoters. It has been found that best results are often achieved by using the Group $V_a$ compound having fromula I in combination with co-promoters such as, for example, metals of Group $I_a$, $II_a$, $III_a$, $IV_b$ or $VI_b$ in the form of finely divided metals or in the form of inorganic or organic compounds. The metal compounds include oxides, hydroxides, halides, oxyhalides, hydrides, carbonyls, alkoxides, nitrates, nitrites, phosphates, phosphites, carboxylates of alkyl, aralkyl or aryl monocarboxylic acids such as acetates, butyrates, stearates and benzoates, chelates, associate compounds and enol-salts. The use of Group $IV_b$ and Group $VI_b$ metal compounds, in particular Zr or Cr compounds such as $ZrOCl_2 . 8H_2O$ or $Cr(CO)_6$ is preferred. The amount of these metal compound promoters may vary within wide limits but amounts of 0.1:1 to 10:1 of mol promoter per gram atom Group VIII noble metal are preferred.

Other compounds which can suitably be used as co-promoter in combination with the Group $V_a$

compound comprise trivalent Group $V_a$ compounds. Examples of these compounds are trivalent nitrogen compounds, for example amines, such as alkyl, cycloalkyl, aryl and aralkyl amines containing up to 30 carbon atoms such as trimethylamine, triethylamine, tetramethylethylene diamine, and especially amines in which the nitrogen atom is part of a heterocyclic ring such as pyrrole, alkyl-substituted pyrroles, pyrrolidine, alkyl-substituted pyrrolidines, pyridine, alkyl-substituted pyridines, piperidines, alkyl-substituted piperidines, pyrimidine, alkyl-substituted alkylpyrimidines, pyrazine, benzatriazole, 1.10-phenanthroline, alkyl-substituted 1.10 phenanthrolines, morpholine and alkyl-substituted morpholines. Preference is given to the use of pyridine and the alkyl-substituted pyridines such as the various picolines for example alpha-picoline.

Further examples of trivalent Group $V_a$ compounds include trivalent phosphorus compounds such as alkyl, cycloalkyl, aryl or alkaryl phosphines having up to 30 carbon atoms, for example trimethylphosphine, triphenylphosphine and tributylphosphine, phosphorus compounds containing two or more phosphine groups, for example tetraphenyl dimethylene diphosphine and tetraphenyl trimethylene diphosphine and heterocyclic phosphorus compounds such as, for example, 1-phenylphospholane and 1-phenylphosphorinane. Also included are trivalent arsenic and antimony compounds such as triphenylarsine, triethylarsine, triphenylstibine or triethylstibine. Another group of suitable trivalent Group $V_a$ compounds are the phosphites such as triphenyl phosphite.

The amount of trivalent Group $V_a$ compound to be used as co-promoter may vary from 0.1:1 to 100:1 mol per gram atom Group VIII metal.

The process according to the present invention can be carried out using a wide range of temperatures. Temperatures up to 300°C can be suitably applied. Preference is given to temperatures in the range of from 50°C to 200°C, most preferred temperatures are in the range between 140°C and 190°C. The process can be carried out using low pressures, for example pressures as low as 5 bar. Pressures in the range of from 20 to 100 bar are preferred. Higher pressures, for example pressures as high as 1000 bar can be applied, but they are generally not economical because of the investment and energy costs involved.

According to the chemical equations presented hereinbefore 1 molecule of carbon monoxide is consumed per molecule methyl acetate in the conversion of methyl acetate into acetic acid anhydride and 2 molecules carbon monoxide and 1 molecule hydrogen in the conversion of methyl acetate into ethylidene diacetate. It has been found, however, that without any substantial disadvantage a wide range of molar ratios of hydrogen to carbon monoxide, for example from 0 to 10 can be applied. As stated earlier the formation of the amount of ethylidene diacetate is largely influenced by the amount of hydrogen present. Thus the process of the invention can flexibly be adapted to a wide variety of product compositions regarding acetic acid anhydride and ethylidene diacetate. For the preparation of acetic acid anhydride preference is given to a molar ratio of hydrogen to carbon monoxide from 0 to 0.1. The preferred range of molar ratios of hydrogen to carbon monoxide for the production of ethylidene diacetate as main product is from 10:1 to 1:10.

The reaction time is not critical and will depend on the temperature and the pressure applied. Reaction times of from 0.25 to 20 hours are sufficient, preference being given to reaction times in the range of from 5 to 15 hours. Shorter or longer reaction times are not excluded, however. The process according to the present invention can be carried out in the liquid phase or in the gaseous phase. Preference is given to the liquid phase which enables a convenient introduction of carbon monoxide and hydrogen into the reaction vessel. If desired carbon monoxide and hydrogen can be introduced together into the reaction vessel.

The process according to the present invention may be carried out in the presence of a solvent. Suitable solvents include carboxylic acids such as acetic acid and propanoic acid, carboxylic acid esters such as methyl acetate (being used as solvent as well as starting material) and cyclic esters such as butyrolacton. Ethers can also be used as solvent, for example dimethyl ether (used both as solvent and as starting material) diethyl ether, methyl-t-butyl ether, diglyme and tetraglyme, and cyclic ethers such as tetrahydrofuran, 1,4-dioxane, 1,3-dioxane and the dioxolanes. Other compounds which can be used as solvent include sulphones and sulphoxides. Examples of such compounds are dimethyl sulphone, diethyl sulphone, methyl ethyl sulphone, methyl butyl sulphone, sulpholane, 2-methylsulpholane, 3-methylsulpholane, 2-methyl-4-butyl sulpholane, dimethyl sulphoxide and diethyl sulphoxide. Preference is given to the use of alkanoic acids especially acetic acid as solvent.

It has been found that the mild conditions used in the process of the invention even tolerate the presence of some water in the reaction medium. Although the presence of water is not preferred, amounts of up to 15%, based on total solvent may be present. The process according to the invention can be carried out batch wise, semi-continuously or continuously. The reaction section may comprise one or more autoclaves or one or more reactor tubes the walls of which are made of or coated with inert materials. The reaction products may be worked up by techniques known in the art. For instance, the reaction product mixture comprising ethylidene diacetate, acetic acid anhydride and unconverted methyl acetate may be subjected to one or more (fractional) distillations to separate the main products ethylidene diacetate and acetic acid anhydride.

The process according to the present invention is also of interest in that it can be integrated with known processes for the production of the starting materials (i.e. methyl acetate and dimethyl ether) and/or for the conversion of the acetic acid anhydride or ethylidene diacetate produced into other products. For instance, when the present process produces ethylidene diacetate, it can be integrated with a process for the

preparation of methyl acetate and acetic acid by carbonylation of methanol and with a process for the preparation of vinyl acetate by decomposition of ethylidene diacetate into vinyl acetate and acetic acid. The acetic acid produced may be internally recycled and converted into methyl acetate by reaction with methanol. So an integrated process can be obtained producing vinyl-acetate from methanol and synthesis gas. The following examples illustrate the invention:

Example I

The experiments 1—8 in this example were carried out using the same technique. The conditions and results of these examples are given in Table A.

A Hastelloy C (Trade mark) 300 ml magnet driven autoclave was charged with methyl acetate, acetic acid and the necessary catalyst components. The vessel was flushed with carbon monoxide and then pressurized with carbon monoxide or carbon monoxide and hydrogen. The autoclave was then heated to a fixed temperature and kept at this temperature during a certain reaction time. The pressure was maintained constant during this reaction time by feeding in carbon monoxide and hydrogen at a ratio corresponding with the partial pressures. After the reaction the reaction mixture was analyzed by gas-liquid chromatography.

The experiments 1—8 show the strong promotive effect of promoters according to the invention on Rh catalysts in absence as well as presence of prior art promoters. The experiments 1, 4 and 7 are comparative experiments not according to the invention.

Example II

The experiments of this example were carried out using the same technique as in the experiments of Example I. The conditions and results are given in Table B. The experiments show that triphenyl phosphine oxide and pyridine oxide promote catalytic systems comprising Pd and prior art promoters. The experiment 9 and 11 are comparative experiments not according to the invention.

Example III

The experiments of this example were carried out using the same technique as for the experiments of Example I. The conditions and results are tabulated in Table C. The experiments show the use of a number of Group $V_a$ compounds as promoters for Rh based catalytic systems. Experiment 14 is not according to the invention and is included as comparative experiment.

TABLE A

| Exp. | Catalyst components mmol: Group VIII metal compound | Promoter | Promoter of the invention | Iodide | Charge, ml: MeOAc | AcOH | CO Partial pressure bar | $H_2$ Partial pressure bar | Reaction temp °C | Reaction time h | Production rate g/g metal/ g iodide/h: EDA | AAA |
|---|---|---|---|---|---|---|---|---|---|---|---|---|
| 1 | $RhCl_3 . 3H_2O$ (0.25) | | | $CH_3I$ 60 | 45 | 5 | 50 | 5 | 160 | 5 | 0.1 | 0.5 |
| 2 | $RhCl_3 . 3H_2O$ (0.25) | | pyridine oxide (4) | $CH_3I$ 60 | 45 | 5 | 50 | 5 | 160 | 5 | 0.2 | 17.6 |
| 3 | $RhCl_3 . 3H_2O$ (0.25) | $ZrOCl_2 . 8H_2O$ (2) | pyridine oxide (4) | $CH_3I$ 60 | 45 | 5 | 50 | 5 | 160 | 5 | 0.9 | 21.4 |
| 4 | $RhCl_3 . 3H_2O$ (0.5) | $ZrOCl_2 . 8H_2O$ (2) | | $CH_3I$ 60 | 40 | 10 | 50 | 0 | 160 | 5 | 0 | 4.7 |
| 5 | $RhCl_3 . 3H_2O$ (0.5) | | $Ph_3P=O$ (4) | $CH_3I$ 60 | 40 | 10 | 50 | 0 | 160 | 5 | 0 | 8.8 |
| 6 | $RhCl_3 . 3H_2O$ (0.5) | $ZrOCl_2 . 8H_2O$ (2) | $Ph_3P=O$ (4) | $CH_3I$ 60 | 40 | 10 | 50 | 0 | 160 | 5 | 0 | 9.9 |
| 7 | $RhCl_3 . 3H_2O$ (0.25) | $Ph_3P$ (0.75) | | $CH_3I$ 60 | 40 | 10 | 30 | 30 | 175 | 5 | 10.5 | 3.3 |
| 8 | $RhCl_3 . 3H_2O$ (0.25) | $Ph_3P$ (0.75) | $Ph_3P=O$ (4) | $CH_3I$ 60 | 40 | 10 | 30 | 30 | 175 | 5 | 11.2 | 10.9 |

EDA=ethylidene diacetate
AAA=acetic acid anhydride
MeOAc=methyl acetate
AcOH=acetic acid

TABLE B

| Exp. | Catalyst components mmol | | | Iodide | Charge, ml | | CO Partial pressure bar | $H_2$ Partial pressure bar | Reaction temp °C | Reaction time h | Production rate g/g metal/ g iodide/h | |
|---|---|---|---|---|---|---|---|---|---|---|---|---|
| | Group VIII metal compound | Promoter | Promoter of the invention | | MeOAc | AcOH | | | | | EDA | AAA |
| 9 | $Pd(OAc)_2$ (1) | $ZrOCl_2 . 8H_2O$ (2) $Ph_3P$ (3) | | $CH_3I$ 60 | 45 | 5 | 50 | 5 | 150 | 5 | 0 | 0.45 |
| 10 | $Pd(OAc)_2$ (1) | $ZrOCl_2 . 8H_2O$ (2) $Ph_3P$ (3) | $Ph_3P{=}O$ (4) | $CH_3I$ 60 | 45 | 5 | 50 | 5 | 150 | 5 | 0 | 0.62 |
| 11 | $Pd(OAc)_2$ (1) | $Cr(CO)_6$ (2) $Ph_3P$ (3) | | $CH_3I$ 60 | 45 | 5 | 30 | 15 | 155 | 5 | 0.6 | 2.3 Cr precipitate after reaction |
| 12 | $Pd(OAc)_2$ (1) | $Cr(CO)_6$ (2) $Ph_3P$ (3) | pyridine oxide (4) | $CH_3I$ 60 | 45 | 5 | 30 | 15 | 155 | 5 | 2.1 | 2.3 |
| 13 | $Pd(OAc)_2$ (1) | $Cr(CO)_6$ (0.5) $Ph_3P$ (3) | pyridine oxide (4) | $CH_3I$ 60 | 45 | 5 | 30 | 15 | 155 | 5 | 3.1 | 0 0.6 acetaldehyde |

EDA=ethylidene diacetate
AAA=acetic acid anhydride
MeOAc=methyl acetate
AcOH=acetic acid

TABLE C

| Exp. | Catalyst components mmol: Group VIII metal compound | Catalyst components mmol: Promoter | Catalyst components mmol: Promoter of the invention | Iodide | Charge, ml: MeOAc | Charge, ml: AcOH | CO Partial pressure bar | $H_2$ Partial pressure bar | Reaction temp °C | Reaction time h | Production rate g/g metal/g iodide/h: EDA | Production rate g/g metal/g iodide/h: AAA |
|---|---|---|---|---|---|---|---|---|---|---|---|---|
| 14 | $RhCl_3 . 3H_2O$ (0.25) | | | $CH_3I$ 60 | 45 | 5 | 20 | 40 | 160 | 5 | 0.6 | 0.5 |
| 15 | $RhCl_3 . 3H_2O$ (0.25) | | $Ph_3P=O$ (4) | $CH_3I$ 60 | 45 | 5 | 30 | 30 | 160 | 5 | 7.4 | 4.3 |
| 16 | $RhCl_3 . 3H_2O$ (0.25) | | $(Ph_3P=O)_2HI_3$ (1) | $CH_3I$ 60 | 45 | 5 | 30 | 15 | 175 | 5 | 7.9 | 16.4 |
| 17 | $RhCl_3 . 3H_2O$ | $ZrOCl_2 . 8H_2O$ | $CH_3-P(=O)-(OCH_3)_2$ (4) | $CH_3I$ 60 | 40 | 5 | 50 | 5 | 160 | 5 | 0.3 | 11.2 |
| 18 | $RhCl_3 . 3H_2O$ (0.25) | $Ph_3P$ (0.75) | $Ph_3P=S$ (4) | $CH_3I$ 60 | 25 | 25 | 20 | 40 | 175 | 5 | 17.0 | 0.4 |
| 19 | $RhCl_3 . 3H_2O$ (0.25) | $Ph_3P$ (0.75) | $Ph_3P=O$ (4) | $CH_3I$ 60 | 25 | 25 | 20 | 40 | 175 | 5 | 15.5 | 0.3 |
| 20 | $RhCl_3 . 3H_2O$ (0.25) | $ZrOCl_2 . 8H_2O$ (2) | $Ph_3P=S$ (4) | $CH_3I$ 60 | 40 | 10 | 50 | 0 | 175 | 2 | 0 | 21.6 |

EDA=ethylidene diacetate
AAA=acetic acid anhydride
MeOAc=methyl acetate
AcOH=acetic acid.

**Claims**

1. A process for the preparation of ethylidene diacetate and/or acetic acid anhydride by reacting methyl acetate and/or dimethyl ether with carbon monoxide or a mixture of carbon monoxide and hydrogen at elevated temperature and pressure in the presence of a homogeneous catalyst system comprising a Group VIII noble metal compound, a bromide or iodide source, characterized in that the reaction is carried out in the presence of a Group Va compound represented by the general formula

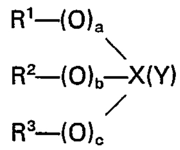

I

in which X is a Group Va element having a valency above 3 selected from N, P, As or Sb; Y is a Group VIa element selected from O, S, or Se and either a, b and c are 0 or 1 and $R^1$, $R^2$ and $R^3$ are similar or dissimilar optionally substituted hydrocarbon groups; or a and b are 0 and c is 0 or 1 and $R^1$ and $R^2$ form together with X a heterocyclic group; or a, b and c are 0 and $R^1$, $R^2$ and $R^3$ form together with X a heterocyclic aromatic group.

2. A process according to claim 1 characterized in that the Group Va compound is represented by the general formula I in which a, b and c are 0, X is P, Y is O or S and $R^1$, $R^2$ and $R^3$ are alkyl groups containing 1—4 carbon atoms or cycloalkyl, aryl or alkaryl groups containing 5—12 carbon atoms.

3. A process according to claim 2 characterized in that the Group Va compound is represented by the general formula I in which Y is O.

4. A process according to claim 3 characterized in that the Group Va compound is triphenyl phosphine oxide or triphenyl phosphine sulphide.

5. A process according to claim 1 characterized in that the Group $V_a$ compound represented by the general formula I is a phosphonate.

6. A process according to claim 1 characterized in that the Group Va compound is represented by the general formula I in which a, b and c are 0, X is N, Y is O and $R^1$, $R^2$ and $R^3$ are alkyl groups containing 1—12 carbon atoms or cycloalkyl, aryl or alkaryl groups containing 5—12 carbon atoms, or $R^1$ and $R^2$ form together with X a heterocyclic group containing 4—12 carbon atoms or $R^1$, $R^2$ and $R^3$ form together with X a heterocyclic aromatic ring containing 5—12 carbon atoms, preferably 5—7 carbon atoms.

7. A process according to claim 6 characterized in that the Group Va compound is pyridine oxide.

8. A process according to claims 1—7 characterized in that one or more Group Ia, IIa, IIIa, IVb or VIb metals or metal compounds are present in the reaction mixture as co-promoter.

9. A process according to claim 8 characterized in that a group IVb metal compounds is present in the reaction mixture.

10. A process according to claim 9 characterized in that the Group $IV_b$ metal compound is a zirconium compound.

11. A process according to claim 8 characterized in that a chromium compounds is present in the reaction mixture.

12. A process according to claim 1 characterized in that a trivalent Group Va compound is present in the reaction mixture as co-promoter.

13. A process according to claim 12 characterized in that the trivalent Group $V_a$ compound is a phosphine.

14. A process according to claim 13 characterized in that the phosphine is triphenyl phosphine.

15. A process according to claims 1—14 characterized in that the Group Va compound represented by the general formula I is present in an ratio of 0.01:1 to 200:1 mole Group Va compound per gram atom Group VIII of noble metal.

16. A process according to claims 1—15 characterized in that the reaction is carried out at a temperature in the range of 50°C to 200°C and preferably between 140°C and 190°C.

17. A process according to claims 1—16 characterized in that the process is carried out using a pressure in the range of from 20 to 100 bar.

18. A process according to claims 1—17 characterized in that the process is carried out in the presence of an alkanoic acid as solvent.

**Patentansprüche**

1. Verfahren zur Herstellung von Ethylidendiacetat und/oder Essigsäureanhydrid durch Umsetzung von Methylacetat und/oder Dimethylether mit Kohlenmonoxid oder einem Gemisch aus Kohlenmonoxid und Wasserstoff bei erhöhter Temperatur und erhöhtem Druck in Gegenwart eines homogenen Katalysatorsystems, umfassend eine Verbindung eines Edelmetalls der Gruppe VIII, eine Bromid- oder Iodidquelle, dadurch gekennzeichnet, daß die Reaktion in Gegenwart einer Verbindung eines Elements der Gruppe Va der allgemeinen Formel

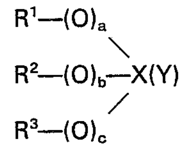

I

durchgeführt wird, wobei X ein Element der Gruppe Va mit einer Wertigkeit von mehr als 3 ist, ausgewählt aus N, P, As oder Sb, Y ein Element der Gruppe VIa ist, ausgewählt aus O, S oder Se, und entweder a, b und c 0 oder 1 sind, und $R^1$, $R^2$ und $R^3$ gleiche oder verschiedene, gegebenenfalls substituierte Kohlenwasserstoff-Gruppen sind oder a und b 0 sind und c 0 oder 1 ist, und $R^1$ und $R^2$ zusammen mit X eine heterocyclische Gruppe bilden oder a, b und c 0 sind und $R^1$, $R^2$ und $R^3$ zusammen mit X eine heterocyclische aromatische Gruppe bilden.

2. Verfahren nach Anspruch 1, dadurch gekennzeichnet, daß die Verbindung des Elements der Gruppe Va angegeben wird durch die allgemeine Formel I, in der a, b und c 0 sind, X=P, Y=O oder S und $R^1$, $R^2$ und $R^3$ (jeweils) Alkyl-Gruppen mit 1 bis 4 Kohlenstoffatomen oder Cycloalkyl-, Aryl- oder Alkaryl-Gruppen mit 5 bis 12 Kohlenstoffatomen bedeuten.

3. Verfahren nach Anspruch 2, dadurch gekennzeichnet, daß die Verbindung des Elements der Gruppe Va angegeben wird durch die allgemeine Formel I, wobei Y gleich 0 ist.

4. Verfahren nach Anspruch 3, dadurch gekennzeichnet, daß die Verbindung des Elements der Gruppe Va Triphenylphosphinoxid oder Triphenylphosphinsulfid ist.

5. Verfahren nach Anspruch 1, dadurch gekennzeichnet, daß die Verbindung des Elements der Gruppe Va der allgemeinen Formel I ein Phosphonat ist.

6. Verfahren nach Anspruch 1, dadurch gekennzeichnet, daß die Verbindung des Elements der Gruppe Va angegeben wird durch die allgemeine Formel I, in der a, b und c 0 sind, X gleich N, Y gleich O ist und $R^1$, $R^2$ und $R^3$ (jeweils) Alkylgruppen mit 1 bis 12 Kohlenstoffatomen oder Cycloalkyl-, Aryl- oder Alkaryl-Gruppen mit 5 bis 12 Kohlenstoffatomen bedeuten oder $R^1$ und $R^2$ zusammen mit X eine heterocyclische Gruppe mit 4 bis 12 Kohlenstoffatomen bilden oder $R^1$, $R^2$ und $R^3$ zusammen einen heterocyclischen aromatischen Ring mit 5 bis 12 Kohlenstoffatomen, vorzugsweise mit 5 bis 7 Kohlenstoffatomen bilden.

7. Verfahren nach Anspruch 6, dadurch gekennzeichnet, daß die Verbindung des Elements der Gruppe Va Pyridinoxid ist.

8. Verfahren nach Anspruch 1 bis 7, dadurch gekennzeichnet, daß ein oder mehrere Metalle oder Verbindungen von Metallen der Gruppen Ia, IIa, IIIa, IVb oder VIb in dem Reaktionsgemisch als Co-Beschleuniger vorhanden sind.

9. Verfahren nach Anspruch 8, dadurch gekennzeichnet, daß eine Verbindung eines Metalls der Gruppe IVb in dem Reaktionsgemisch vorhanden ist.

10. Verfahren nach Anspruch 9, dadurch gekennzeichnet, daß die Verbindung des Metalls der Gruppe IVb eine Zirkonium-Verbindung ist.

11. Verfahren nach Anspruch 8, dadurch gekennzeichnet, daß eine Chromverbindung in dem Reaktionsgemisch vorhanden ist.

12. Verfahren nach Anspruch 1, dadurch gekennzeichnet, daß eine 3-wertige Verbindung eines Elements der Gruppe Va in dem Reaktionsgemisch als Co-Beschleuniger vorhanden ist.

13. Verfahren nach Anspruch 12, dadurch gekennzeichnet, daß die 3-wertige Verbindung des Elements der Gruppe Va ein Phosphin ist.

14. Verfahren nach Anspruch 13, dadurch gekennzeichnet, daß das Phosphin Triphenylphosphin ist.

15. Verfahren nach Anspruch 1 bis 14, dadurch gekennzeichnet, daß die Verbindung des Elements der Gruppe Va, die durch die allgemeine Formel I angegeben wird, in einem Verhältnis von 0,01:1 bis 200:1 Mol-Verbindung eines Elements der Gruppe Va pro Gramm Atom Edelmetall VIII vorhanden ist.

16. Verfahren nach Anspruch 1 bis 15, dadurch gekennzeichnet, daß die Reaktion bei einer Temperatur im Bereich von 50 bis 200°C, vorzugsweise zwischen 140 und 190°C, durchgeführt wird.

17. Verfahren nach Anspruch 1 bis 16, dadurch gekennzeichnet, daß das Verfahren unter einem Druck im Bereich von 20 bis 100 bar durchgeführt wird.

18. Verfahren nach Anspruch 1 bis 17, dadurch gekennzeichnet, daß das Verfahren in Gegenwart einer Alkansäure als Lösungsmittel durchgeführt wird.

**Revendications**

1. Un procédé pour la préparation de diacétate d'éthylidène et/ou d'anhydride d'acide acétique par réaction d'acétate de méthyle et/ou d'oxyde de méthyle avec l'oxyde de carbone ou un mélange d'oxyde de carbone et d'hydrogène à température et pression élevées en présence d'un système catalytique homogène comprenant un composé d'un métal noble du groupe VIII, une source de bromure ou d'iodure, caractérisé en ce que la réaction est conduite en présence d'un composé du groupe $V_a$ représenté par la formule générale

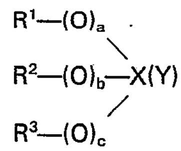

I

où X est un élément du groupe $V_a$ ayant une valence de plus de 3 choisi parmi N, P, As ou Sb; Y est un élément du groupe $VI_a$ choisi parmi O, S ou Se et a, b et c sont 0 ou 1 et $R^1$, $R^2$ et $R^3$ sont des groupes d'hydrocarbures éventuellement substitués identiques ou différents; ou a et b sont 0 et c est 0 ou 1 et $R^1$ et $R^2$ forment ensemble avec X un groupe hétérocyclique; ou a, b et c sont 0 et $R^1$, $R^2$ et $R^3$ forment ensemble avec X un groupe aromatique hétérocyclique.

2. Un procédé selon la revendication 1, caractérisé en ce que le composé du groupe $V_a$ est représenté par la formule générale I dans laquelle a, b et c sont 0, X est P, Y est O ou S et $R^1$, $R^2$ et $R^3$ sont des groupes alcoyle contenant 1—4 atomes de carbone ou des groupes cycloalcoyle, aryle ou alcaryle contenant 5—12 atomes de carbone.

3. Un procédé selon la revendication 2, caractérisé en ce que le composé du groupe $V_a$ est représenté par la formule générale I dans laquelle Y est O.

4. Un procédé selon la revendication 3, caractérisé en ce que le composé du groupe $V_a$ est l'oxyde de triphénylphosphine ou le sulfure de triphénylphosphine.

5. Un procédé selon la revendication 4, caractérisé en ce que le composé du groupe $V_a$ représenté par la formule générale I est un phosphonate.

6. Un procédé selon la revendication 1, caractérisé en ce que le composé du groupe $V_a$ est représenté par la formule générale I dans laquelle a, b et c sont 0, X est N, Y est O et $R^1$, $R^2$ et $R^3$ sont des groupes alcoyle contenant 1—12 atomes de carbone ou des groupes cycloalcoyle, aryle ou alcaryle contenant 5—12 atomes de carbone, ou $R^1$ et $R^2$ forment ensemble avec X un groupe hétérocyclique contenant 4—12 atomes de carbone ou $R^1$, $R^2$ et $R^3$ forment ensemble avec X un noyau aromatique hétérocyclique contenant 5—12 atomes de carbone, de préférence 5—7 atomes de carbone.

7. Un procédé selon la revendication 6, caractérisé en ce que le composé du groupe $V_a$ est l'oxyde de pyridine.

8. Un procédé selon les revendications 1—7, caractérisé en ce qu'un ou plusieurs métaux ou composés de métaux des groupes Ia, IIa, IIIa, IVb ou VIb sont présents dans le mélange réactionnel comme co-promoteur.

9. Un procédé selon la revendication 8, caractérisé en ce qu'un composé d'un métal du groupe $IV_b$ est présent dans le mélange réactionnel.

10. Un procédé selon la revendication 9, caractérisé en ce que le composé d'un métal du groupe $IV_b$ est un composé du zirconium.

11. Un procédé selon la revendication 8, caractérisé en ce qu'un composé du chrome est présent dans le mélange réactionnel.

12. Un procédé selon la revendication 1, caractérisé en ce qu'un composé du groupe $V_a$ trivalent est présent dans le mélange réactionnel comme co-promoteur.

13. Un procédé selon la revendication 12, caractérisé en ce que le composé du groupe $V_a$ trivalent est une phosphine.

14. Un procédé selon la revendication 13, caractérisé en ce que la phosphine est la triphénylphosphine.

15. Un procédé selon les revendications 1—14, caractérisé en ce que le composé du groupe $V_a$ représenté par la formule générale I est présent à raison de 0,01 à 200 moles de composé du groupe $V_a$ par atome-gramme de métal noble du groupe VIII.

16. Un procédé selon les revendications 1—15, caractérisé en ce que la réaction est conduite à une température comprise entre 50°C et 200°C et de préférence entre 140°C et 190°C.

17. Un procédé selon les revendications 1—16, caractérisé en ce qu'on met en oeuvre le procédé en utilisant une pression comprise entre 20 et 100 bars.

18. Un procédé selon les revendications 1—17, caractérisé en ce qu'on met en oeuvre le procédé en présence d'un acide alcanoïque comme solvant.